# EUROPEAN PATENT SPECIFICATION

(11) **EP 1 238 965 B1**
(45) Date of publication and mention of the grant of the patent: **08.06.2005**
(21) Application number: 01301839.5
(22) Date of filing: 28.02.2001
(51) Int. Cl.: C07C 213/00, C07C 213/02

(54) **A process for the preparation of 1-(2-dimethylamino-(4-methoxyphenyl)-ethyl)cyclohexanol**
Verfahren zur Herstellung von 1-(2-Dimethylamino-(4-methoxyphenyl)-ethyl)cyclohexanol
Procédé de préparation du 1-(2-diméthylamino-(4-méthoxyphényl)-éthyl)cyclohexanol

(43) Date of publication of application: 11.09.2002
(73) Proprietor: Council of Scientific and Industrial Research, New Delhi 110 001 (IN)
(72) Inventor: Chavan, Subhash Prataprao, Pune 411 008, Maharashtra (IN); Kamat, Subhash Krishnaji, Pune 411 008, Maharashtra (IN); Sivadasan, Latha, Pune 411 008, Maharashtra (IN); Balakrishnan, Kamalam, Pune 411 008, Maharashtra (IN); Khobragade, Dushant Anandrao, Pune 411 008, Maharashtra (IN); Thottapillil, Ravindranathan, Pune 411 008, Maharashtra (IN); Gurjar, Mukund Keshao, Pune 411 008, Maharashtra (IN); Kalkote, Uttam Ramrao, Pune 411 008, Maharashtra (IN)
(74) Representative: Manaton, Ross Timothy

(56) References cited:
- EP-A- 0 112 669
- US-A- 2 462 736
- FRISTAD, W E ET AL: "Manganese(III) gamma-Lactone Annulation with substituted Acids" JOURNAL OF ORGANIC CHEMISTRY, vol. 50, no. 17, 1985, pages 3143-3148, XP000996322
- YARDLEY J P ET AL.: "2-Phenyl-2-(1-hydroxycycloalkyl)ethylamin e Derivatives : Synthesis and Antidepressant Activity" JOURNAL OF MEDICINAL CHEMISTRY, vol. 33, no. 10, 1990, pages 2899-2905, XP000996354

## Description

### Field of the invention

The present invention relates to a one pot process for the preparation of 1-[2-dimethylamino-(4-methoxyphenyl)ethyl]cyclohexanol of formula 1. 1-[2-dimethylamino-(4-methoxyphenyl)ethyl]cyclohexanol of formula 1 is commonly known as Venlafaxine and also as effexor. More particularly, the present invention relates to a one pot synthesis of compound of formula 1 from 1 [Cyano(4-methoxyphenyl)methyl]-cyclo-hexanol having formula 2 which is in turn prepared by a single step conversion comprising the condensation of 4-methoxyphenylacetonitrile with cyclohexanone in the presence of a convenient base like sodium hydroxide.

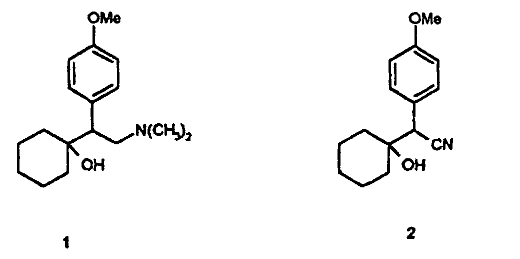

### Background of the invention

Venlafaxine having formula 1 and pharmaceutically acceptable salts thereof are important antidepressants of the central nervous system developed by Wyeth-Ayerat & Company in 1993 [Zhou Jinpei, Zhang Huibin, Huang Xuezhe Huang Wenlong J, China Pharm. Univ. 1999, 30(4) P.249-50].

In the prior art [Husbands et al. U.S. Patent No.4, 535, 186 (1985)] compound of the formula 1 is prepared by reaction of p-methoxy phenyl acetonitrile at -78°C, with cyclohexanone under the influence of n-butyl lithium, following available methods [Sauvetre et al. Tetrahedron 34 2135 (1978)] followed by reduction under high pressure using Rhodium on alumina as the catalyst obtained through Lou-c-kart reaction. Symmetrical N-methylation is accomplished via modified Eschweiler - Clarkes procedure employing formalin, formic acid and a large excess of water as illustrated by Tilford et al [J.A.C.S. 76, 2431 (1954)]. Alternatively, the procedure of Borch and Hassid using sodium cyano borohydride and formaldehyde is employed. Fristed, W.E. et al *Manganese (III) γ-Lactone Annulation with Substituted Acids* (Am. Chem. Soc. [1985] 4143-3148) mentions that dihydro-3-[(*N*,*N*-dimethylamino) methyl]-5-octyl-2(3*H*)-furanone may be synthesised from the corresponding α-cyano lactone by treatment with formaldehyde in ethanol under a hydrogen atmosphere, using a Raney nickel catalyst. US 2,462,736 (published in 1949) relates to the synthesis of N-alkyl substituted alkanolamines from simple aldehyde and ketone cyanhydrins, the examples relating to the reaction of formaldehyde with either formaldehyde cyanhydrin or acetone cyanhydrin. Neither document relates to the compounds the subject of the present invention.

In another prior art [Robin Gerald Shepherd UK Patent No GB 2 227 743 A (1990)] the condensation of 4-methoxyphenylacetonitrile with cyclohexanone is accomplished by the use of lithiumdiisopropylamide in hydrocarbon solvents like hexane toluene or cyclohexane at ambient temperature thereby improving the yield to 79% and further reduction of 2 to the amine followed by protection of the amine to the required compound of formula 1.

In yet another prior art [Zhou Jinpei, Zhang Huibin, Huang Xuezhen, Huang Wenlong J, China Pharm. Univ. 1999, 30(4) P.249-50], anisole is acylated to the chloroacetyl derivative and aminated using N,N Dimethylamine, the carbonyl group of this compound reduced to alcohol using KBH₄ and converted to the bromo derivative using PBr₃ which in turn when reacted with Mg and then cyclohexanone underwent a Grignard reaction to provide Venlafaxine.

The use of reagents like butyllithium and lithiumdiisopropylamide, Rhodium on alumina and chloroacetylchloride poses severe drawbacks because of their hazardous nature. More over the solvents used are also hazardous and inconvenient in the large- scale preparation of compound of formula 1.

### Objects of the invention

The object of the present invention is to provide a simple and convenient method of synthesis of venlafaxine using easily available raw materials.

### Summary of the invention

Accordingly, the present invention relates to a process for the preparation of 2-[dimethylamino-(4-methoxyphenyl)ethyl]-cyclohexanol of formula 1 which comprises reducing 1-[cyano(4-methoxyphenyl)-methyl] cyclohexanol of formula 2 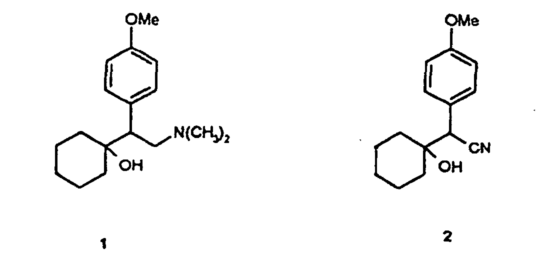 with a formylating agent in a protic solvent in the presence of a catalyst at a temperature in the range between 30-60°C for a time period in the range of 6-16 hours at a pressure in the range of 100 to 400 psi (approximately 6.89 × 10⁵ to 2.76 × 10⁶ N.m⁻²) of hydrogen, removing the catalyst by filtration, isolating and purifying the compound of formula 1.

In one embodiment of the invention the unreacted compound of formula 2 is recycled.

In one embodiment of the invention, the formylating agent comprises 35% formalin.

In another embodiment of the invention, the protic solvent comprises methyl alcohol.

In a further embodiment of the invention, the catalyst is Raney nickel.

In another embodiment of the present invention, compound of formula 2 is prepared by treatment of cyclohexanone with 4-methoxy phenyl acetonitrile in the presence of base by the process described in copending patent application number EP-A-1,238,967

In another embodiment of the present invention, the Raney nickel used is in the ratio 1:1, 2:1 1 and 3:1 (w/v) as that of the starting material.

In still another embodiment of the present invention, the pressure is 200 psi (approximately 1.38 × 10⁶N.m⁻²) of hydrogen.

In yet another embodiment of the present invention the reaction time is 10 hours.

In a further embodiment of the invention, the unreacted starting material is fully recovered and recycled.

### Detailed description of the invention

The process of the present invention is described by the following examples, which are illustrative only and should not be construed as limit to the scope of the reaction in any manner.

### Example 1

To a solution of 1[Cyano (4-methoxyphenyl)methyl]cyclohexanol having formula (2) (5.0 parts, 0.02 mole) in methanol (100 parts) was added formalin (35%soln, 25 parts) and Raney nickel (5 ml, settled material). The mixture was hydrogenated under pressure (200 psi approximately 1.38 × 10⁶N.m⁻²) at 60°C for 6 hrs. The reaction was removed, filtered, the Raney nickel washed with methanol, (4 x 25 parts) the combined filtrates concentrated to an oily residue. It was then dissolved in ethyl acetate (100 parts) and partitioned between 10% dil HCl. The aqueous layer was washed with ethyl acetate, basified to pH 10 using 25% aqueous sodium hydroxide solution, saturated with sodium chloride and extracted with ethyl acetate, after washing with brine (2 x 25 parts) and drying over Na₂SO₄ was concentrated on rotaevaporator to get a bright white solid m.p. 74-6°C. Yield 1.6 parts (28%). The acid insoluble portion after washing and drying was concentrated to get the unreacted nitrile (3 parts, 60%). ¹HNMR CDCl₃) δ(ppm): 7.32-6.98 (4H, q, p-substituted aromatic) 3.78 (3H, s, OCH₃) 3.64 (2H, m, CH₂ N(CH₃)₂ 3.06 (1H, m, CH-CH₂N-(CH₃) 2.74 (6H, s, N(CH₃)₂; 1.38 (10H, Br m, aliphatic cyclohexyl).

### Example 2

To a solution of 1[Cyano(4-methoxyphenyl)methyl]cyclohexanol having formula (2) (5.0 parts, 0.02 mole) in methanol (100 parts) was added formalin (35%soln, 25 parts) and Raney nickel (5 ml, settled material). The mixture was hydrogenated under pressure (200 psi approximately 1.38 × 10⁶ N.m⁻²) at 30°C for 16 hrs. The reaction was removed, filtered, the Raney nickel washed with methanol, (4 x 25 parts) the combined filtrates concentrated to an oily residue. It was then dissolved in ethyl acetate (100 parts) and partitioned between 10% dil HCl. The aqueous layer was washed with ethyl acetate, basified to pH 10 using 25% aqueous sodium hydroxide solution, saturated with sodium chloride and extracted with ethyl acetate, after washing with brine (2 x 25 parts) and drying (Na₂SO₄) was concentrated on a rotaevaporator to get a bright white solid m.p. 74-6°C. Yield 0.85 parts (15%). The acid insoluble portion after washing and drying was concentrated to get the unreacted nitrile 2 -3 parts, 60%).

### Example 3

To a solution of 1 [Cyano(4-methoxyphenyl)methyl]cyclohexanol having formula (2) (5.0 parts, 0.02 mole) in methanol (100 parts) was added formalin (35%soln, 25 parts) and Raney nickel (1.25 ml, settled material). The mixture was hydrogenated under pressure (200 psi approximately 1.38 x 10⁶ N.m⁻²) at 60°C for 16 hrs. The reaction was removed, filtered, the Raney nickel washed with methanol, (4 x 25 parts) and the combined filtrates concentrated to an oily residue. It was then dissolved in ethyl acetate (100 parts) and partitioned between 10% dil HCI. The aqueous layer was washed with ethyl acetate, basified to pH 10 using 25% aqueous sodium hydroxide solution, saturated with sodium chloride and extracted into ethyl acetate, after washing with brine (2 x 25 parts) and drying (Na₂SO₄) was concentrated on a rotaevaporator to get a bright white solid m.p. 74-6°C. Yield 0.85 parts (15%). The acid insoluble portion after washing and drying was concentrated to get the unreacted nitrile 2 (3.2 parts, 64%) .

### Example 4

To a solution of 1 [Cyano(4-methoxyphenyl)methyl]cyclohexanol having formula (2) (5.0 parts, 0.02 mole) in methanol (100 parts) was added formalin (35%soln, 25 parts) and Raney nickel (2.5 ml, settled material). The mixture was hydrogenated under pressure (400 psi approximately 2.76 × 10⁶N.m⁻²) at 60°C for 10 hrs. The reaction was removed, filtered, the Raney nickel washed with methanol, (4 x 25 parts) the combined filtrates concentrated to an oily residue. It was then dissolved in ethyl acetate (100 parts) and partitioned between 10% dil HCI. The aqueous layer was washed with ethyl acetate, basified to pH 10 using 25% aqueous sodium hydroxide solution, saturated with sodium chloride and reextracted into ethyl acetate, after washing with brine (2 x 25 parts) and drying (Na₂SO₄) was concentrated on a rotaevaporator to get a bright white solid m.p. 74-6°C. Yield 1.6 parts,30%. The ethyl acetate portion after washing with water (2x20 parts) and drying was concentrated to get the unreacted nitrile 2 (3.2 parts, 64%).

## Claims

1. A process for the preparation of 2-[dimethylamino-(4-methoxyphenyl)ethyl]-cyclohexanol of formula 1 which comprises reducing 1-[cyano(4-methoxyphenyl)-methyl] cyclohexanol of formula 2 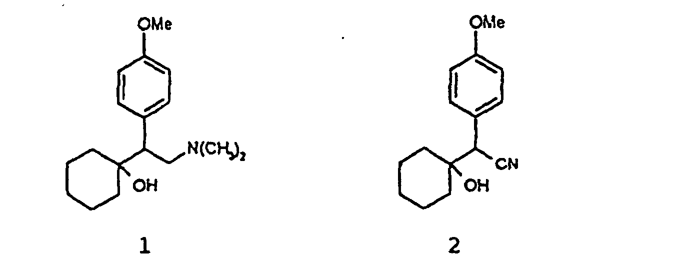 with a formylating agent in a protic solvent in the presence of a catalyst at a temperature in the range between 30-60°C for a time period in the range of 6-16 hours at a pressure in the range of 100 to 400 psi (approximately 6.89 x 10⁵ to 2.76 x 10⁶N.M⁻²) of hydrogen, removing the catalyst by filtration, isolating and purifying the compound of formula 1.

2. A process as claimed in claim 1 wherein the formylating agent comprises 3 5 % formalin.

3. A process as claimed in claim 1 or claim 2, wherein the protic solvent comprises methyl alcohol.

4. A process as claimed in any of claims 1 to 3 wherein the catalyst used is Raney nickel.

5. A process as claimed in claim 4 wherein the Raney nickel used is in the ratio 1:1 to 3:1 (w/v) to the starting material.

6. A process as claimed in any preceding claim wherein the pressure is 200 psi of hydrogen.

7. A process as claimed in any preceding claim wherein the reaction time is 10 hours.

8. A process as claimed in any preceding claim wherein the unreacted starting material is fully recovered and recycled.

## Patentansprüche

1. Verfahren zur Herstellung von 2-[Dimethylamino-(4-methoxyphenyl)ethyl]cyclohexanol der Formel 1, bei dem man 1-[Cyano(4-methoxyphenyl)methyl]cyclohexanol der Formel 2 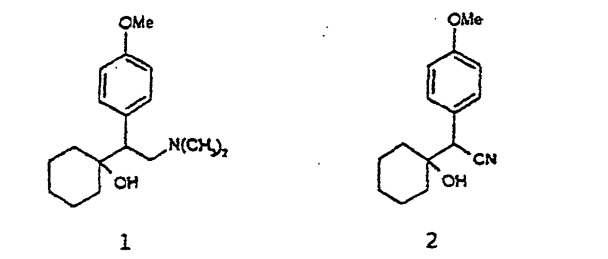 mit einem Formylierungsmittel in einem protischen Lösungsmittel in Gegenwart eines Katalysators bei einer Temperatur im Bereich zwischen 30-60°C über einen Zeitraum im Bereich von 6-16 Stunden bei einem Wasserstoffdruck im Bereich von 100 bis 400 psi (ungefähr 6,89 x 10⁵ bis 2,76 x 10⁶ N.M⁻²) reduziert, den Katalysator abfiltriert und die Verbindung der Formel 1 isoliert und reinigt.

2. Verfahren nach Anspruch 1, bei dem man als Formylierungsmittel 35%iges Formalin verwendet.

3. Verfahren nach Anspruch 1, bei dem man als protisches Lösungsmittel Methylalkohol verwendet.

4. Verfahren nach einem der Ansprüche 1 bis 3, bei dem man als Katalysator Raney-Nickel verwendet.

5. Verfahren nach Anspruch 4, bei dem man das Raney-Nickel in einem Verhältnis von 1:1 bis 3:1 (w/v) zum Edukt verwendet.

6. Verfahren nach einem der vorhergehenden Ansprüche, bei dem der Wasserstoffdruck 200 psi beträgt.

7. Verfahren nach einem der vorhergehenden Ansprüche, bei dem die Reaktionszeit 10 Stunden beträgt.

8. Verfahren nach einem der vorhergehenden Ansprüche, bei dem man das nicht umgesetzte Edukt vollständig zurückgewinnt und zurückführt.

## Revendications

1. Procédé de préparation de 2-[diméthylamino-(4-méthoxyphényl)éthyl]cyclohexanol de formule 1 qui comprend la réduction du 1-[cyano(4-méthoxyphényl)méthyl]cyclohexanol de formule 2 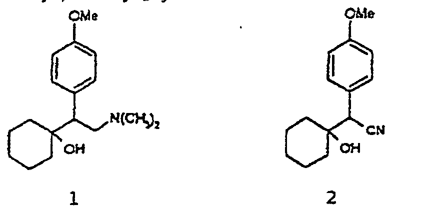 avec un agent de formylation dans un solvant protique en présence d'un catalyseur à une température dans la gamme comprise entre 30-60°C pendant une durée dans la gamme de 6-16 heures sous une pression dans la gamme de 100 à 400 psi (environ 6,89 x 10⁵ à 2,76 x 10⁶ N.m⁻²) d'hydrogène, l'élimination du catalyseur par filtration, l'isolement et la purification du composé de formule 1.

2. Procédé selon la revendication 1 dans lequel l'agent de formylation comprend 35% de formaline.

3. Procédé selon la revendication 1 ou 2, dans lequel le solvant protique comprend de l'alcool méthylique.

4. Procédé selon l'une quelconque des revendications 1 à 3 dans lequel le catalyseur employé est du nickel de Raney.

5. Procédé selon la revendication 4 dans lequel le nickel de Raney utilisé l'est dans une proportion de 1:1 à 3:1 (en poids/volume) par rapport au composé de départ.

6. Procédé selon l'une quelconque des revendications précédentes dans lequel la pression est de 200 psi d'hydrogène.

7. Procédé selon l'une quelconque des revendications précédentes dans lequel la durée de la réaction est de 10 heures.

8. Procédé selon l'une quelconque des revendications précédentes dans lequel le composé de départ n'ayant pas réagi est entièrement récupéré et recyclé.
